# EUROPÄISCHE PATENTSCHRIFT

(11) **EP 4 193 967 B1**
(45) Veröffentlichungstag und Bekanntmachung des Hinweises auf die Patenterteilung: **17.09.2025**
(21) Anmeldenummer: 21213244.3
(22) Anmeldetag: 08.12.2021
(51) Int. Cl.: A61F 2/44, A61F 2/30

(54) **MODULARER STÜTZKÄFIG**
MODULAR SUPPORT CAGE
CAGE D'APPUI MODULAIRE

(43) Veröffentlichungstag der Anmeldung: 14.06.2023
(73) Patentinhaber: PR Spine GbR, 55122 Mainz (DE)
(72) Erfinder: Pippan, Mathias, 55122 Mainz (DE); Reith, Michael, 55130 Mainz (DE)
(74) Vertreter: Stolmár & Partner Patentanwälte PartG mbB

(56) Entgegenhaltungen:
- US-A1- 2018 071 102
- US-B2- 6 562 074

## Beschreibung

### Gebiet der Erfindung

Die Erfindung betrifft einen modularen Stützkäfig für das Stützen von Wirbelelementen.

### Stand der Technik

Bei Schäden an der Bandscheibe werden zur Stützung der Wirbelelemente sogenannte Wirbelkäfige verwendet. Diese werden zum Stützen der Wirbelsäule zwischen die einzelnen Wirbelelemente statt der geschädigten Bandscheibe eingesetzt. Um eine möglichst natürliche Stellung der einzelnen Wirbelelemente zueinander zu gewährleisten, soll die Lordose der Wirbelsäule nachgebildet werden. Da die Wirbelelemente an der Vorderseite einen größeren Abstand als an der Rückseite aufweisen, sollte ein solcher Wirbelkäfig ebenfalls an der Vorderseite dicker ausgebildet sein, damit der natürliche Abstand der Wirbelelemente erhalten bleibt. In diesem Fall können die Wirbelkäfige aber nicht von hinten durch den Rücken, sondern müssen durch die Bauchdecke operativ eingesetzt werden. Denn da die Vorderseite des Wirbelkäfigs mit einem größeren Abstand ausgebildet ist und die Wirbelelemente auf der Rückseite nicht auf diesen Abstand auseinandergeschoben werden können, passt der Wirbelkäfig nicht durch die Lücke zwischen den benachbart liegenden Wirbelelementen. Alternativ kann für das Einsetzen von der Rückseite ein kleinerer Wirbelkäfig verwendet werden, mit dem dann allerdings der ideale Abstand zwischen den Wirbeln und die gewünschte Lordose nicht erreicht werden können.

Ein solcher ventraler Wirbelkäfig ist bspw. in der US 6,562,074 B2 beschrieben. Dort ist ein einstellbares Knochenfusionsimplantat offenbart, das eine erste Platte mit einer Innenfläche mit einer Vielzahl von beabstandeten ersten Stützelementen umfasst, die von dieser hervorstehen. Jedes Stützelement hat eine Vielzahl von hervorstehenden Zähnen. Eine zweite Platte weist eine Innenfläche mit einer Vielzahl von beabstandeten zweiten Stützelementen auf, die von der zweiten Platte hervorstehen. Jedes zweite Stützelement weist mindestens einen darauf ausgebildeten Zahn oder ein Einstellloch auf. Ein Bereich der Vielzahl von Zähnen jedes ersten Stützelements greift mechanisch mit dem mindestens einen Zahn oder einem Einstellloch eines entsprechenden zweiten Stützelements ein, so dass die erste Platte und die zweite Platte selektiv getrennt werden können, um dazwischen eine Kammer zu bilden. Ein Verstärkungselement ist zwischen der ersten Platte und der zweiten Platte angeordnet, so dass das Aufbringen einer Druckkraft zwischen der ersten Platte und der zweiten Platte eine Kompression auf das Verstärkungselement ausübt.

Die US 2018/0071102 A1 offenbart ein montierbares Implantat aus zwei oder mehr Elementen, die in einer bestimmten Entfernung voneinander positionierbar sind, die mit einem oder mehreren biokompatiblen Stiften zusammengehalten werden, um eine Transplantateinheit zu bilden.

### Darstellung der Erfindung

Aufgabe der Erfindung ist es daher einen Wirbelkäfig bereitzustellen, der unabhängig von der Lordose minimalinvasiv von der Rückseite des Patienten implantiert werden kann. Diese Aufgabe wird gelöst durch einen modularen Stützkäfig nach Anspruch 1.

Ein erfindungsgemäßer modularer Stützkäfig für das Stützen von Wirbelelementen, umfasst einen distalen Stützabschnitt, der zumindest zwei gegenüberliegende und zueinander bewegbare Endplatten aufweist, wobei eine Endplatte jeweils einen Kontaktbereich, der nach außen gerichtet ist und für den Kontakt mit dem zu stützenden Element ausgebildet ist, und jeweils einen Lagerbereich umfasst, der zu einer gegenüberliegenden Endplatte gerichtet ist und einen proximalen Stützabschnitt, der zumindest zwei nach außen gerichtete Kontaktbereiche und ein Lagerelement aufweist, das zwischen die Lagerbereiche der Endplatten einfügbar ist und diese lagert, so dass die Endplatten in einer vorbestimmten Position gehalten sind. Durch die Aufteilung in getrennte vordere und hintere Elemente ist es möglich den distalen Stützabschnitt, der weiter vom Anwender weg entfernt ist, zuerst durch den schmalen Spalt zwischen den Wirbelelementen in seine Position zu schieben und erst danach den proximalen Stützabschnitt zu positionieren. Dazu werden die Endplatten des ersten Stützabschnitts bspw. mit einem Werkzeug auseinandergedrückt oder sonst irgendwie voneinander weg bewegt, damit die Kontaktbereiche der Endplatten eine Neigung und Position aufweisen, in der die Wirbelelemente in der natürlichen Lordose gestützt werden. Dann wird das Lagerelement zwischen die voneinander beabstandeten Endplatten geschoben, so dass diese in der eingestellten Position gehalten werden. Daher benötigt der distale Stützabschnitt beim Einschieben zwischen die Wirbel nicht mehr Platz als der proximale Stützabschnitt und der modulare Stützkäfig kann für jede Lordose von der Rückseite her implantiert werden. Das Lagerelement ist daher in der Endposition zwischen den ersten Kontaktbereichen angeordnet. Ferner bedeutet "distal" eine Position vom Anwender des Stützkäfigs weg gerichtet und "proximal" eine Position näher am Anwender, wobei der Anwender die Person ist, die den Stützkäfig handhabt. Ferner bedeutet "modular" im Erfindungssinn eine getrennte Aufteilung in einen vorderen (distalen) und einen hinteren (proximalen) Teil und nicht eine Trennung der Stützabschnitte in oben und unten (auch wenn eine Trennung oben/unten in der erfindungsgemäßen Ausführungsform zumindest für das distale Stützelement ebenfalls vorgesehen ist, um die Lordose einstellen zu können)

Vorzugsweise sind die Kontaktbereiche der distalen und proximalen Stützabschnitte aneinander ausgerichtet und bilden insbesondere eine gemeinsame Ebene aus. Das heisst, die zueinander gerichteten Kanten der ersten und zweiten Stützabschnitte liegen stufenlos aneinander an und weisen insbesondere auch eine gleiche Steigung auf, weisen also keinen Knick in der Fläche auf (knicklos). Dadurch wird eine gleichmäßige Stützung der Wirbelelemente gesichert und eine ausreichend große Stützfläche des Gesamtelementes sichergestellt.

Die Kontaktbereiche und das Lagerelement des proximalen Stützabschnitts sind vorzugsweise einstückig ausgebildet. Das erleichtert die Handhabung und erhöht die Stabilität und die Sicherheit beim Einschieben des proximalen Stützabschnitts.

Ferner kann zumindest eine der zwei Endplatten mit einer sich verjüngenden Dicke ausgebildet sein, so dass der Kontaktbereich eine insbesondere kontinuierlich verlaufende Neigung aufweist, bevorzugt haben aber alle Endplatten eine solche sich verjüngende Dicke. Ebenfalls vorzugsweise gilt die sich verjüngenden Dicke auch für den proximalen Stützabschnitt. Durch eine Ausbildung mit einer sich verjüngenden Dicke wird die gewünscht Lordose zuverlässig bereitgestellt. Eine massive Ausbildung oder eine Ausbildung mit einer Trabekelstruktur kann die Stabilität der Stützabschnitte verbessern.

Die Endplatten weisen vorzugsweise einen Anschlag auf, insbesondere einen Aufnahmeeingriff an ihrem distalen Ende, der als Anschlag für das Lagerelement dient. Dadurch kann die Endposition des Lagerelements präzise bestimmt werden und eine versehentliche Fehlpositionierung kann zuverlässig vermieden werden. Ein Aufnahmeeingriff sichert die Endplatten zusätzlich noch gegen ein versehentliches Abheben vom Lagerelement ab.

Bevorzugt weisen das Lagerelement und die Lagerbereiche an der Schnittstelle zwischen distalem und proximalen Stützabschnitt Stufenelemente auf. Dadurch wird ein seitliches Verschwenken der Stützabschnitte in der Endposition vermieden und die korrekte Ausrichtung sichergestellt. Ein solcher Stufenabschnitt ist insbesondere auch als Anschlag für die Einschubrichtung verwendbar. Ferner kann auch am distalen Ende der Kontaktbereiche ein zweiter Aufnahmeeingriff ausgebildet sein, in den das proximale Ende der Endplatten eingreift und damit den Zusammenhalt der Endplatte am Lagerelement absichert.

Die zumindest zwei nach außen gerichteten Kontaktbereiche des proximalen Stützabschnitts umfassen vorzugsweise jeweils zwei beabstandete Flächen und die zumindest zwei nach außen gerichteten Kontaktbereiche der distalen Stützabschnitte sind bevorzugt jeweils U-förmig ausgebildet. Dadurch ist es auf einfache Weise möglich, die Werkzeuge für die beiden Stützabschnitte zu handhaben, ohne dass sich diese gegenseitig behindern.

Vorzugsweise umfasst der Stützkäfig eine Verankerung eines Lagerelements und eines Stützabschnitts, so dass der Stützkäfig zuverlässiger verwendet werden kann. Bevorzugt umfasst der Stützkäfig zumindest einen Stützabschnitt, der zumindest zwei gegenüberliegende und zueinander bewegbare Stützplatten aufweist, wobei eine Stützplatte jeweils einen Kontaktbereich, der nach außen gerichtet und für den Kontakt mit dem zu stützenden Element ausgebildet ist, und jeweils einen Lagerbereich umfasst, der zu einer gegenüberliegenden Stützplatte gerichtet ist, und ein Lagerelement, der zwischen die Stützplatten einfügbar ist und diese lagert, so dass die Stützplatten in einer vorbestimmten Position gehalten sind. Das Lagerelement und die Stützabschnitte des Stützkäfigs oder des modularen Stützkäfigs weisen an dem distalen Endbereich ferner zueinander komplementäre Rastelemente auf, die in der Endposition des Lagerelements derart zusammenwirken, dass das Lagerelement bewegungsfest mit den Stützabschnitten verbunden ist.

Die Rastelemente (bspw. Federstift und Bohrung) können selbstverständlich auch mit der vorhergehenden Ausführungsform verwendet werden, die die distalen und proximalen Stützabschnitte aufweist, so dass dort ebenfalls das Lagerelement und die Stützabschnitte des Stützkäfigs oder des modularen Stützkäfigs an dem distalen Endbereich ferner zueinander komplementäre Rastelemente aufweisen können, die in der Endposition des Lagerelements derart zusammenwirken, dass das Lagerelement bewegungsfest mit den Stützabschnitten verbunden ist.

Mit dem Rastelement wird auf einfache Weise sichergestellt, dass nach dem Einstellen der Lordose und dem Einschieben des Lagerelements zum Fixieren der Stützabschnitte das Lagerelement nicht mehr bewegt werden kann.

Die Rastelemente sind vorzugsweise als federgetriebener Stift und als Aufnahme ausgebildet. Diese Ausgestaltung ermöglicht eine zuverlässige Auslösung des Rastelements, wenn die Endposition erreicht ist. Der federgetriebene Stift ist bevorzugt am Lagerelement und die Aufnahme ist vorzugsweise an den Lagerbereichen der Stützabschnitte ausgebildet. Dadurch ist sichergestellt, dass das Rastelement für das Auslösen des Rastelements gut zugänglich und einfach erreichbar ist.

Die Endplatten des distalen Stützabschnitts können eine Werkzeugaufnahme aufweisen, die insbesondere als Bohrung ausgebildet ist. Eine solche Werkzeugaufnahme erleichtert die Handhabung beim Einsetzen des proximalen Stützabschnitts. Die Bohrung ist vorzugsweise kreisrund, wodurch die Ausrichtung des einzusetzenden Werkzeugs unerheblich und das Einstecken in die Werkzeugaufnahme erleichtert ist. Sie kann aber auch oval oder vieleckig (bspw. drei-, vier-, sechseckig) sein, wodurch die Ausrichtung des Werkzeugs sichergestellt ist.

### Kurze Beschreibung der Figuren

Figur 1 zeigt eine isometrische Ansicht des modularen Stützkäfigs in einem zusammengebauten Zustand;
Figuren 2a und 2b zeigen isometrische Ansichten des modularen Stützkäfigs mit Hilfswerkzeugen beim Einschieben des proximalen Stützabschnitts in den distalen Stützabschnitt;
Figur 3 zeigt einen Querschnitt durch das Rastelement im distalen Stützabschnitt;

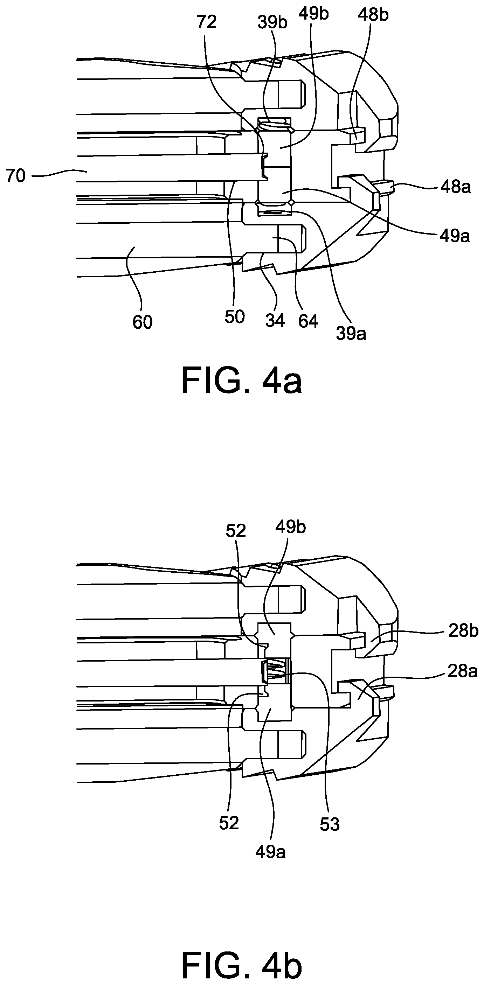

Figuren 4a und 4b zeigen einen Längsschnitt durch das Rastelement im distalen Stützabschnitt im offenen und im eingerasteten Zustand; und
Figuren 5a und 5b zeigen isometrische Ansichten auf die proximale Seite (Rückseite) der Stützabschnitte.

### Beschreibung der bevorzugten Ausführungsformen

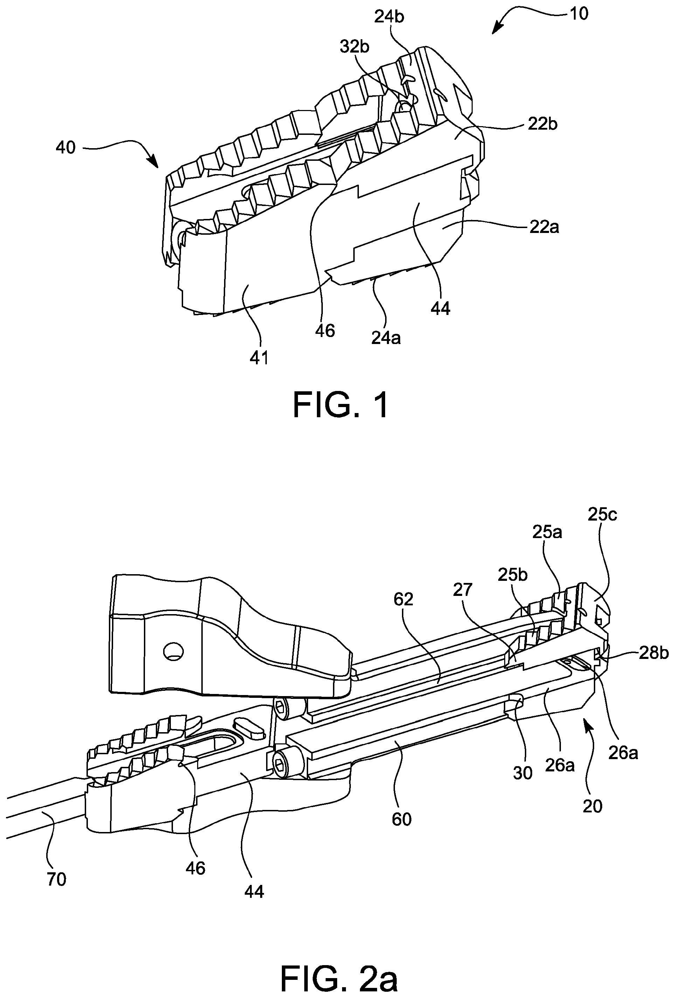

In Figur 1 ist ein zusammengesteckter modularer Stützkäfig 10 in einer isometrischen Ansicht dargestellt. Der Stützkäfig umfasst einen distalen Stützabschnitt 20 und einen proximalen Stützabschnitt 40.

Der distale Stützabschnitt 20 ist in der vorliegenden Ausführungsform zweiteilig ausgebildet und umfasst zumindest zwei Endplatten 22a, 22b. Diese Endplatten 22a, 22b weisen jeweils eine Kontaktfläche 24a, 24b auf, die nach außen gerichtet ist (in Figur 1 nach oben bzw. nach unten) und die im Einsatz mit den benachbarten Wirbelelementen in Kontakt steht. Auf der den Kontaktflächen 22a, 22b gegenüberliegenden Seite sind Lagerbereiche 26a, 26b vorgesehen, die zur Abstützung an einem Lagerelement 44 dienen, so dass die Endplatten 22a, 22b vom Lagerelement in der Endposition gehalten werden. Die Endplatten 22a, 22b sind vorzugsweise einstückig und weiter bevorzugt U-förmig ausgebildet, wobei sich die zwei beabstandeten Seitenarme 25a, 25b proximal, also zum Anwender hin erstrecken und der Verbindungssteg 25c, der beide Seitenarme 25a, 25b verbindet, am distalen Ende der Endplatten 22a, 22b vorgesehen ist. Die Endplatten 22a, 22b sind mit einer sich verjüngenden Dicke ausgebildet, so dass die Kontaktfläche 24a, 24b sich vom proximalen Ende bis zum distalen Ende hin (beim Verbindungssteg 25c) neigt, wobei die Endplatten 22a, 22b am proximalen Ende dünner sind und zum distalen Ende hin dicker werden. Die Neigung der Kontaktflächen 24a, 24b kann aber auch dadurch erreicht werden, dass nur eine der Endplatten 22a, 22b mit einer sich verjüngenden Dicke ausgebildet ist, wobei die Verjüngung dann entsprechend stärker ausgebildet sein sollte.

Die Endplatten 22a, 22b weisen bevorzugt einen Anschlag 30 auf, der beispielsweise als einfaches Stufenelement ausgebildet sein kann. Ferner kann am distalen Ende der Endplatten 22a, 22b ein Aufnahmeeingriff 28a, 28b vorgesehen sein, der nach dem Eingriff mit dem später beschriebenen Lagerelement 44 die Endplatte 22a, 22b fest auf dem Lagerelement 44 hält. Dieser Aufnahmeeingriff 28a, 28b wird bevorzugt auch als Anschlag verwendet.

Ferner weisen die Endplatten auf der nach innen gerichteten Seite (gegenüber den Kontaktflächen) Lagerbereiche 26a, 26b auf, die mit dem Lagerelement 44 zusammenwirken. An diesen Lagerbereichen 26a, 26b sind bevorzugt Führungsstufen vorgesehen, die in Figur 2a zu erkennen sind. Diese wirken mit den Stufenabschnitten 62 in den Haltewerkzeugen 60 zusammen und führen so die Haltewerkzeuge 60 sicher. Ferner können auch Werkzeugaufnahmen 32a, 32b an den Endplatten 22a, 22b ausgebildet sein, in die die Haltewerkzeuge 60 einführbar sind und die insbesondere zusammen mit den Führungsstufen ein sicheres Halten der Endplatten 22a, 22b gewährleisten können. Die Werkzeugaufnahme 32a, 32b kann im Prinzip beliebig ausgebildet sein, bspw. oval oder mehreckig, eine kreisrunde Bohrung ist jedoch die bevorzugte Ausbildung der Werkzeugaufnahme.

Der proximale Stützabschnitt 40 weist einen Kontaktabschnitt 41 auf, an dem die obere und die untere Kontaktflächen 42a, 42b ausgebildet sind, die im Einsatz mit den Wirbelelementen in Kontakt stehen. Ferner umfasst der proximale Stützabschnitt 40 ein Lagerelement 44, das sich in distaler Richtung erstreckt, das zwischen den Endabschnitten 22a, 22b des distalen Stützabschnitts 20 platziert wird und mit den Lagerbereichen 24a, 24b jeweils oben und unten in Kontakt steht. Das Lagerelement 44 ist vorzugsweise einstückig mit dem Kontaktabschnitt 41 ausgebildet. Ferner ist es möglich, dass das Lagerelement 44 mit einer sich verjüngenden Dicke ausgebildet ist, so dass das Lagerelement 44 zum distalen Ende hin dünner wird. Diese Verjüngung wird dann mit einer entsprechenden Verdickung der Endplatten 22a, 22b ausgeglichen um trotzdem die korrekte Lordose zu erhalten. Die Verjüngung dient dazu, dass das Auseinanderdrücken der Endplatten 22a, 22b mittels des Haltewerkzeugs 60 durch das Einschieben des Lagerelementes 44 unterstützt wird.

Der proximale Stützabschnitt 40 ist vorzugsweise U-förmig ausgebildet, so dass die Kontaktflächen 42a, 42b jeweils als zwei voneinander beabstandete Flächen auf einem Seitenarm vorgesehen sind und am distalen Ende ein Verbindungssteg diese Seitenarme verbindet. In der Mitte des Verbindungsstegs erstreckt sich das Lagerelement 44 in distaler Richtung. Am proximalen Ende des Kontaktabschnitts kann ebenfalls ein Verbindungssteg 56 ausgebildet sein, der die beiden Seitenarme verbindet und stabilisiert. Der Verbindungssteg 56 weist ferner eine Aussparung oder eine Durchgangsbohrung 54 auf, durch die das Einsetzwerkzeug 70 an die Werkzeugaufnahme 50 geführt werden kann.

Ferner kann der proximale Stützabschnitt 40 auch einen Aufnahmeeingriff 46 aufweisen, der vorzugsweise am distalen Ende der Kontaktflächen 44a, 44b vorgesehen ist, an dem sich vorzugsweise die Kontaktflächen 44a, 44b und die Kontaktflächen 24a, 24b treffen. Hier ist der Aufnahmeeingriff 46 als dreieckiger Hinterschnitt ausgebildet, in den ein dreieckiger Vorsprung 27 der Kontaktflächen 24a, 24b des distalen Stützabschnitts 20 eingesteckt und dieser so fest auf dem Lagerelement 44 gehalten wird.

Für das Einsetzwerkzeug 70 weist der proximale Stützabschnitt 40 an seinem proximalen Ende eine Werkzeugaufnahme 62 auf, die im Prinzip wie die Werkzeugaufnahme im distalen Stützabschnitt 20 ausgebildet sein kann (kreisrund, oval, mehreckig). In einer bevorzugten Ausgestaltung ist sie als eine Bohrung ausgebildet. Die Werkzeugaufnahme 50 reicht vorzugsweise bis an einen Rastmechanismus und ermöglicht so dem Einsetzwerkzeug 70 mit dem Rastmechanismus zusammenzuwirken. In diesem Zusammenhang ist auch die oben beschriebene U-Förmigkeit ein großer Vorteil, denn diese ermöglicht durch den Abstand zwischen den Seitenarmen, dass die Werkzeugaufnahme 70 des Verbindungsstegs möglichst ortsnah zum später beschriebenen Rastmechanismus angeordnet ist und der Rastmechanismus bei der Vorbereitung leichter gehandhabt werden kann.

Am distalen Ende des proximalen Stützabschnitts 40 sind weiter vorzugsweise Eingriffsvorsprünge 48a, 48b ausgebildet, die mit den Aufnahmeeingriffen 28a, 28b zusammenwirken, um beide Stützabschnitte 20, 40 aneinander zu halten.

Die Kontaktflächen 22a, 22b, 42a, 42b der Stützabschnitte weisen vorzugsweise ein Muster auf. Dieses Muster kann beispielsweise Querrippen oder andere Erhebungen wie einfache Vorsprünge umfassen. In der gezeigten Ausgestaltung ist das Muster sind als dreieckige (gezackte) Rippen dargestellt, die insbesondere als Stufen ausgebildet sind, die an die Lordose angepasst sind, so dass die Oberseite der Stufen fast waagrecht ausgebildet ist und die Vorderseiten fast senkrecht (mit einer Neigung kleiner als 10° Grad).

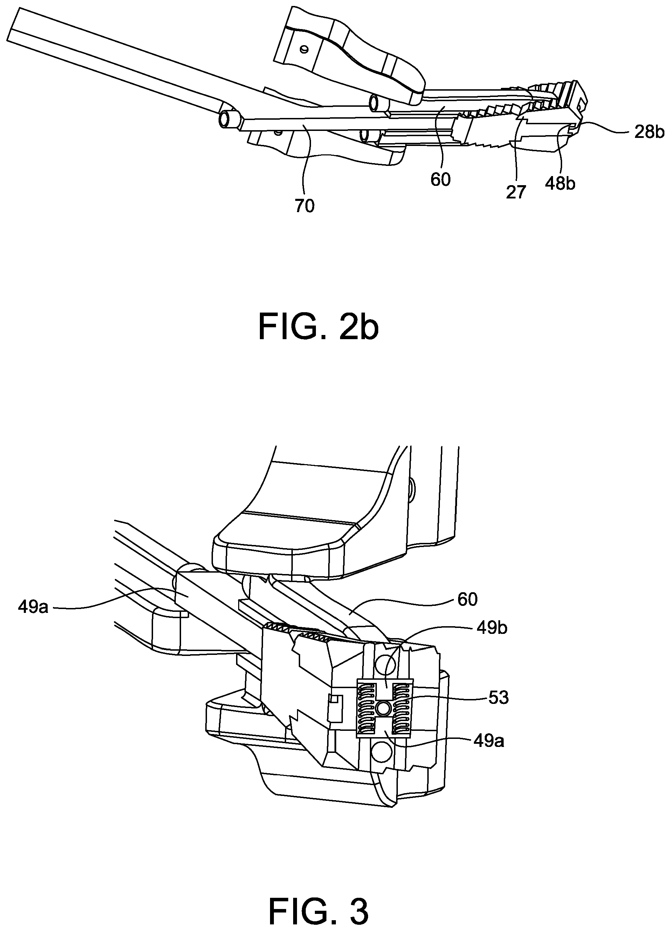

In den Figuren 2a und 2b ist dargestellt, wie der proximale und der distale Stützabschnitt ineinander geschoben werden. Zuerst werden die Haltewerkzeuge 60 mit dem Stufenabschnitt 62 an die entsprechende Führungsschiene der Endplatten 22a, 22b gelegt und dann in die Werkzeugaufnahme 34a, 34b eingesteckt. Der distale Stützabschnitt wird dann zwischen den Wirbeln platziert. Die in den Figuren gezeigten Haltewerkzeuge 60 können bspw. die Enden einer Zange sein, mit denen die Endplatten 22a, 22b des distalen Stützabschnitts 20 auseinandergedrückt werden können. in Figur 2a sind die Endplatten 22a, 22b schon in einer voneinander beabstandeten Position gezeigt, in der die richtige Lordose eingestellt ist. Ferner ist zu sehen, wie das Einsteckwerkzeug 70 bereitgestellt ist, um in den proximalen Stützabschnitt 40 eingesetzt zu werden.

In Figur 2b ist dann der proximale Stützabschnitt 40 in Richtung des distalen Stützabschnitts 20 verschoben worden, so dass sich das Lagerelement 44 zwischen den Endplatten 22a, 22b befindet und deren Lagerbereiche 26a, 26b kontaktiert und stützt. Beim Zusammenschieben haben sich, falls vorhanden, die Eingriffsvorsprünge 48a, 48b am distalen Ende des Stützkäfigs in die Aufnahmeeingriffe 28a, 28b bewegt. Ebenso ist dies an der Verbindungsstelle der Kontaktflächen 24a, 24b, 44a, 44b geschehen, wo sich der Aufnahmeeingriff 46 über den Vorsprung 27 geschoben hat.

In dieser Position kann bei dem modularen Stützkäfig ein Rastmechanismus vorgesehen sein, der das Lagerelement 44 fest und insbesondere auch irreversibel mit den Endplatten 22a, 22b verbindet. Ein solcher Rastmechanismus kann auch bei einem nicht-modularen Stützkäfig (keine Trennung in distal und proximal) verwendet werden, der dann zwei Stützabschnitte aufweist, die sowohl oben als auch unten einstückig aus einem proximalen und einem distalen Stützabschnitt ausgebildet sind. Zusätzlich gibt es dann ein Lagerelement, das zwischen die Endplatten des distalen Stützabschnitts eingeschoben wird und diese lagert, analog zum Lagerelement 44, das den distalen Stützabschnitt 20 lagert. Diese Ausführungsform ist in den Figuren nicht gezeigt, der Rastmechanismus funktioniert aber in der gleichen Weise wie der nachfolgend erläuterte Rastmechanismus für den modularen Stützkäfig.

Der Rastmechanismus umfasst vorzugsweise zwei Rastelemente, die hier bevorzugt als federgetriebener Rastblock 49a, 49b und als Rastaufnahme 39a, 39b ausgebildet sind, wobei der Begriff Rastblock auch eine Ausbildung als Raststift oder Rastkugel umfasst. In der vorliegenden Ausbildung sind der federgetriebene Rastblock 49 im Lagerelement 44 und die Rastaufnahme 39 in den Endplatten 22a, 22b angeordnet. Der federgetriebene Rastblock 49 kann aber auch umgekehrt in den Endplatten 22a, 22b und die Rastaufnahme im Lagerelement 44 angeordnet sein. Der Rastblock 49a, 49b ist mit einer Feder vorgespannt, die den Rastblock nach in Richtung Endplatte 22a, 22b drückt. Hier ist für beide Rastblöcke 49a, 49b eine oder mehrere Federn 53 vorgesehen, die beide Rastblöcke 49a, 49b gleichzeitig in die jeweils entgegengesetzte Richtung vorspannen können. Insbesondere kann tatsächlich nur eine Feder vorgesehen sein. Die Rastblöcke 49a, 49b werden also innerhalb des Lagerelements 44 gehalten bis die Rastaufnahme erreicht ist und werden dann von der Feder 53 in die Rastaufnahme gedrückt.

Damit die Rastblöcke 49a, 49b aber nicht umständlich mit einem Werkzeug in dem Lagerelement gehalten werden müssen, bis die Rastblöcke 49a, 49b unter den Endplatten 22a, 22b eingeklemmt sind, sind ferner vorzugsweise Sperrvertiefungen 52 vorgesehen in den Rastblöcken 49a, 49b vorgesehen, die mit den Haltevorsprüngen 72 des Einsetzwerkzeugs 70 zusammenwirken. Hier werden die Rastblöcke 49a, 49b von außen gegen die Feder zusammengedrückt und dann die Haltevorsprünge 72 des Einsetzwerkzeugs 70 erst in die Werkzeugaufnahme 50 des proximalen Stützabschnitts 40 und dann in die in die Sperrvertiefungen 52 eingeführt. Dadurch dass die Federn die Rastblöcke 49a, 49b nach außen drücken, verklemmen sich die Sperrvertiefungen 52 mit den Haltevorsprüngen 82 und verbessern deren Halt. Erst wenn sich der proximale Stützabschnitt 40 tatsächlich in der Endposition befindet, liegen die Rastblöcke 49a, 49b unter den entsprechenden Aufnahmen 39a, 39b. Dann wird das Einsetzwerkzeug aus der Werkzeugaufnahme 50 herausgezogen und damit auch die Haltevorsprünge 72 aus den Sperrvertiefungen 52 entfernt. Dadurch drücken die vorgespannten Federn in die Rastaufnahmen 39a, 39b und die Rastblöcke 49a, 49b fixieren das Lagerelement 44 in den Endplatten, so dass ein Herausziehen nicht mehr möglich ist ohne die Rastblöcke aus den Rastaufnahmen zu entfernen.

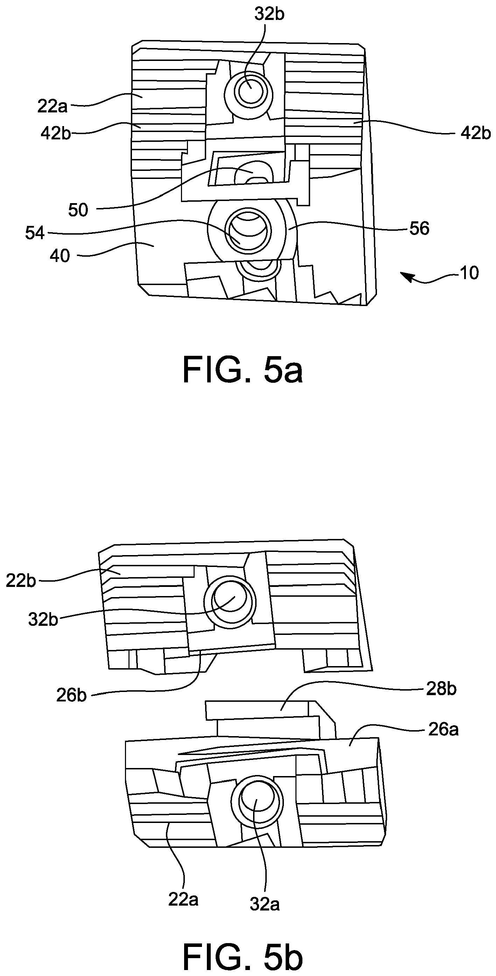

Figur 5a zeigt eine Ansicht auf einen zusammengesteckten modularen Stützkäfig 10. Aus dieser Ansicht sind die Werkzeugaufnahmen 32a, 32b des distalen Stützabschnitts und die Werkzeugaufnahme 50 des proximalen Stützabschnitts 40. Die Werkzeugaufnahme 50 des proximalen Stützabschnitts 40 ist am Lagerelement 44 vorgesehen.

Vorzugsweise ist eine weitere Bohrung 54 oder eine Aussparung 54 an einer proximalen Verstärkungsstrebe 56 vorgesehen, die die Kontaktflächen 42a, 42b mit den gegenüberliegenden Kontaktflächen 42a, 42b verbindet und so stabilisiert.

In Figur 5b ist das distale Stützelement 20 so dargestellt, dass man von hinten auf die Werkzeugaufnahmen 32a, 32b sehen kann. Auch der Aufnahmeeingriff 28a ist gut zu erkennen.

### Bezugszeichenliste

modularer Stützkäfig 10
distaler Stützabschnitt 20
Endplatte 22a, 22b
Kontaktfläche 24a, 24b
Seitenarm 25a, 25b
Verbindungssteg 25c
Lagerbereich 26a, 26b
Vorsprung 27
Aufnahmeeingriff 28a, 28b
Anschlag 30
Werkzeugaufnahme 32a, 32b
Rastaufnahme 39a, 39b
proximaler Stützabschnitt 40
Kontaktabschnitt 41
Kontaktfläche 42a, 42b
Lagerelement 44
Aufnahmeeingriff 46
Feder 47a, 47b
Eingriffsvorsprung 48a, 48b
federgetriebener Rastblock 49a, 49b
Werkzeugaufnahme 50
Sperrvertiefungen 52
Feder 53
Durchgangsbohrung 54
Verbindungssteg 56
Haltewerkzeug 60
Stufenabschnitt 62
Steckvorsprung 64
Einsetzwerkzeug 70
Haltevorsprung 72

## Patentansprüche

1. Modularer Stützkäfig (10) für das Stützen von Wirbelelementen, umfassend:
einen distalen Stützabschnitt (20), der zumindest zwei gegenüberliegende und zueinander bewegbare Endplatten (22a, 22b) aufweist, wobei eine Endplatte (22a, 22b) jeweils eine nach außen gerichtete Kontaktfläche (24a, 24b), die für den Kontakt mit dem zu stützenden Element ausgebildet ist, und jeweils einen Lagerbereich (24a, 24b) aufweist, der zu einer gegenüberliegenden Endplatte (22a, 22b) gerichtet ist; und
einen proximalen Stützabschnitt (40), der zumindest zwei nach außen gerichtete Kontaktflächen (42a, 42b) und ein Lagerelement (44) aufweist, das zwischen die Lagerbereiche (24a, 24b) der Endplatten (22a, 22b) einfügbar ist und diese lagert, so dass die Endplatten (22a, 22b) in einer vorbestimmten Position gehalten sind.

2. Modularer Stützkäfig (10) nach Anspruch 1, bei dem die Kontaktflächen (24a, 24b, 42a, 42b) der distalen und proximalen Stützabschnitte (20, 40) aneinander ausgerichtet sind.

3. Modularer Stützkäfig (10) nach einem der vorhergehenden Ansprüche, bei dem die Kontaktflächen (42a, 42b) und das Lagerelement (44) des proximale Stützabschnitts (40) einstückig ausgebildet sind.

4. Modularer Stützkäfig (10) nach einem der vorhergehenden Ansprüche, bei dem die zumindest zwei Endplatten (22a, 22b) und/oder zumindest teilweise der proximale Stützabschnitt (40) mit einer sich verjüngenden Dicke ausgebildet sind, so dass die Kontaktflächen (24a, 24b, 42a, 42b) geneigt sind.

5. Modularer Stützkäfig (10) nach einem der vorhergehenden Ansprüche, bei dem die Endplatten (22a, 22b) einen Anschlag für das Lagerelement (44) aufweisen.

6. Modularer Stützkäfig (10) nach einem der vorhergehenden Ansprüche, bei dem das Lagerelement (44) und die Lagerbereiche (24a, 24b) an der Schnittstelle zwischen distalem und proximalen Stützabschnitt (20, 40) Stufenelemente (30) aufweisen.

7. Modularer Stützkäfig (10) nach einem der vorhergehenden Ansprüche, bei dem die zumindest zwei nach außen gerichteten Kontaktbereiche des proximalen Stützabschnitts jeweils zwei beabstandete Flächen aufweisen und die zumindest zwei nach außen gerichteten Kontaktbereiche der distalen Stützabschnitte jeweils U-förmig ausgebildet sind.

8. Modularer Stützkäfig (10) nach einem der vorhergehenden Ansprüche, wobei das Lagerelement (44) und die Stützplatten des Stützkäfigs oder die Endplattem (22a, 22b) des modularen Stützkäfigs (10) an einem distalen Endbereich ferner zueinander komplementäre Rastelemente (39a, 39b, 49a, 49b) aufweisen, die in der Endposition des Lagerelements (44) derart zusammenwirken, dass das Lagerelement (44) bewegungsfest mit den Stützabschnitten oder Endplatten (22a, 22b) verbunden ist.

9. Stützkäfig oder modularer Stützkäfig (10) nach Anspruch 8, wobei die Rastelemente als federgetriebener Rastblock (49a, 49b) und als Rastaufnahme (39a, 39b) ausgebildet sind.

10. Stützkäfig oder modularer Stützkäfig (10) nach Anspruch 9, bei dem der federgetriebene Rastblock (49a, 49b) am Lagerelement (44) und die Rastaufnahme (39a, 39b) an den Lagerbereichen (26a, 26b) der Endplatten (22a, 22b) ausgebildet sind.

11. Stützkäfig oder modularer Stützkäfig (10) nach einem der vorhergehenden Ansprüche, wobei die Endplatten des distalen Stützabschnitts und/oder der proximale Stützabschnitt eine Werkzeugaufnahme (34a, 34b, 50) aufweisen.

## Claims

1. Modular support cage (10) for supporting vertebral elements, comprising:
a distal support section (20) having at least two end plates (22a, 22b) which are opposite and movable relative to each other, one end plate (22a, 22b) each having an outwardly directed contact surface (24a, 24b) designed for contact with the element to be supported, and each having a bearing region (24a, 24b) directed toward an opposite end plate (22a, 22b); and
a proximal support section (40) having at least two outwardly directed contact surfaces (42a, 42b) and a bearing element (44) which is insertable between the bearing areas (24a, 24b) of the end plates (22a, 22b) and bears these so that the end plates (22a, 22b) are held in a predetermined position.

2. Modular support cage (10) according to claim 1, wherein the contact surfaces (24a, 24b, 42a, 42b) of the distal and proximal support sections (20, 40) are aligned with each other.

3. Modular support cage (10) according to one of the preceding claims, wherein the contact surfaces (42a, 42b) and the bearing element (44) of the proximal support section (40) are designed in a single piece.

4. Modular support cage (10) according to one of the preceding claims, wherein the at least two end plates (22a, 22b) and/or at least part of the proximal support section (40) are designed with a tapered thickness so that the contact surfaces (24a, 24b, 42a, 42b) are inclined.

5. Modular support cage (10) according to one of the preceding claims, wherein the end plates (22a, 22b) have a stop for the bearing element (44).

6. Modular support cage (10) according to one of the preceding claims, wherein the bearing element (44) and the bearing areas (24a, 24b) have step elements (30) at the interface between the distal and proximal support sections (20, 40).

7. Modular support cage (10) according to one of the preceding claims, wherein the at least two outwardly directed contact areas of the proximal support section each have two spaced surfaces and the at least two outwardly directed contact areas of the distal support sections are each U-shaped.

8. Modular support cage (10) according to one of the preceding claims, wherein the bearing element (44) and the support plates of the support cage or the end plates (22a, 22b) of the modular support cage (10) further have complementary locking elements (39a, 39b, 49a, 49b) at a distal end region (39a, 39b, 49a, 49b) which, in the end position of the bearing element (44), interact in such a way that the bearing element (44) is fixed in terms of movement with the support sections or end plates (22a, 22b).

9. Support cage or modular support cage (10) according to claim 8, wherein the locking elements are designed as a spring-driven locking block (49a, 49b) and as a locking receptacle (39a, 39b).

10. Support cage or modular support cage (10) according to claim 9, wherein the spring-driven locking block (49a, 49b) is formed on the bearing element (44) and the locking receptacle (39a, 39b) is formed on the bearing areas (26a, 26b) of the end plates (22a, 22b).

11. Support cage or modular support cage (10) according to one of the preceding claims, wherein the end plates of the distal support section and/or the proximal support section have a tool receptacle (34a, 34b, 50).

## Revendications

1. Cage de soutien modulaire (10) pour soutenir des éléments vertébraux, comprenant :
une partie de soutien distale (20) qui présente au moins deux plaques d'extrémité (22a, 22b) opposées et mobiles l'une par rapport à l'autre, une plaque d'extrémité (22a, 22b) comportant chacune une surface de contact (24a, 24b) orientée vers l'extérieur, qui est conçue pour entrer en contact avec l'élément à soutenir, et chacune une surface de palier (24a, 24b) qui est orientée vers une plaque d'extrémité opposée (22a, 22b) ; et
une partie de support proximale (40) qui comporte au moins deux surfaces de contact (42a, 42b) orientées vers l'extérieur et un élément de palier (44) qui peut être inséré entre les surfaces de palier (24a, 24b) des plaques d'extrémité (22a, 22b) et qui supporte celles-ci de telle sorte que les plaques d'extrémité (22a, 22b) soient maintenues dans une position prédéterminée.

2. Cage de support modulaire (10) selon la revendication 1, dans laquelle les surfaces de contact (24a, 24b, 42a, 42b) des parties de support distale et proximale (20, 40) sont alignées les unes avec les autres.

3. Cage de support modulaire (10) selon l'une des revendications précédentes, dans laquelle les surfaces de contact (42a, 42b) et l'élément de palier (44) de la partie de support proximale (40) sont formés d'un seul tenant.

4. Cage de support modulaire (10) selon l'une des revendications précédentes, dans laquelle les au moins deux plaques d'extrémité (22a, 22b) et/ou au moins une partie de la section de support proximale (40) sont conçues avec une épaisseur qui diminue progressivement, de sorte que les surfaces de contact (24a, 24b, 42a, 42b) sont inclinées.

5. Cage de support modulaire (10) selon l'une des revendications précédentes, dans laquelle les plaques d'extrémité (22a, 22b) présentent une butée pour l'élément de palier (44).

6. Cage de support modulaire (10) selon l'une des revendications précédentes, dans laquelle l'élément de palier (44) et les surfaces de palier (24a, 24b) présentent des éléments en gradins (30) à l'intersection entre la partie de support distale et la partie de support proximale (20, 40).

7. Cage de support modulaire (10) selon l'une des revendications précédentes, dans laquelle les au moins deux zones de contact orientées vers l'extérieur de la partie de support proximale présentent chacune deux surfaces espacées et les au moins deux zones de contact orientées vers l'extérieur des parties de support distales sont chacune réalisées en forme de U.

8. Cage de support modulaire (10) selon l'une des revendications précédentes, dans laquelle l'élément de palier (44) et les plaques de support de la cage de support ou les plaques d'extrémité (22a, 22b) de la cage de support modulaire (10) présentent, sur une zone d'extrémité distale, des éléments d'encliquetage complémentaires les uns aux autres (39a, 39b, 49a, 49b) qui, dans la position finale de l'élément de palier (44), coopèrent de telle sorte que l'élément de palier (44) est relié de manière immobile aux sections de support ou aux plaques d'extrémité (22a, 22b).

9. Cage de support ou cage de support modulaire (10) selon la revendication 8, les éléments d'encliquetage étant réalisés sous forme de bloc d'encliquetage à ressort (49a, 49b) et de logement d'encliquetage (39a, 39b).

10. Cage de support ou cage de support modulaire (10) selon la revendication 9, dans laquelle le bloc d'encliquetage actionné par ressort (49a, 49b) est formé sur l'élément de palier (44) et le logement d'encliquetage (39a, 39b) sur les surfaces de palier (26a, 26b) des plaques d'extrémité (22a, 22b).

11. Cage de support ou cage de support modulaire (10) selon l'une des revendications précédentes, les plaques d'extrémité de la partie de support distale et/ou de la partie de support proximale comportant un logement d'outil (34a, 34b, 50).
